# FASCICULE DE BREVET EUROPEEN

(11) **EP 1 019 327 B1**
(45) Date de publication et mention de la délivrance du brevet: **15.02.2006**
(21) Numéro de dépôt: 98944027.6
(22) Date de dépôt: 21.09.1998
(51) Int. Cl.: C02F 1/70, C07D 249/14, C12P 17/10, C12R 1/10

(54) **PROCEDE D'ELIMINATION D'UN COMPOSE HETEROCYCLIQUE AZOTE, OU AROMATIQUE, DANS UN EFFLUENT**
VERFAHREN ZUR ELIMINIERUNG EINER STICKSTOFF ENTHALTENDEN HETEROCYCLISCHEN ODER AROMATISCHEN VERBINDUNG AUS ABWASSER
METHOD FOR ELIMINATING A NITROGENATED HETEROCYCLIC, OR AROMATIC, COMPOUND IN AN EFFLUENT

(30) Priorité: 22.09.1997 FR 9711757
(43) Date de publication de la demande: 19.07.2000
(73) Titulaire: COMMISSARIAT A L'ENERGIE ATOMIQUE, 75015 Paris (FR); The Centre National de la Recherche Scientifique, 75794 Paris Cedex 16 (FR)
(72) Inventeur: LE CAMPION, Laurence, F-91350 Grigny (FR); OUAZZANI, Jamal, F-91300 Massy (FR)
(74) Mandataire: Audier, Philippe André
(86) Numéro de dépôt international: PCT/FR1998/002011
(87) Numéro de publication internationale: WO 1999/015465

(56) Documents cités:
- WO-A-96/25365

## Description

### Domaine technique de l'invention

La présente invention se rapporte à un procédé d'élimination d'un composé hétérocyclique azoté, ou aromatique, comportant au moins un groupe nitro. Le procédé de l'invention consiste à convertir le, au moins un, groupe nitro en groupe amino, au moyen d'un agent réducteur. L'agent réducteur peut être un micro-organisme capable de réduire ledit, au moins un, groupe nitro en groupe amino, ou de l'hydrogène en présence d'un catalyseur Pd/C.

La présente invention se rapporte également à un procédé de synthèse de 5-amino-1,2,4-triazole-3-one, à partir de 5-nitro-1,2,4-triazole-3-one par ledit procédé de conversion.

La présente invention se rapporte également à une souche de type Bacillus licheniformis capable de convertir un (des) groupe(s) nitro de composés hétérocycliques azotés, ou aromatiques, en groupe(s) amino.

Le composé hétérocyclique azoté peut être par exemple une triazine ou un triazole. Ces composés constituent une famille hétérogène de molécules azotées dont plusieurs sont utilisées dans l'industrie et l'agriculture. Bien que la toxicité de ces composés n'a pas toujours été mise en évidence, relâchés dans la nature ils peuvent cependant constituer une source de pollution.

Un exemple d'un tel composé est l'ONTA, où 1,2-dihydro-5-nitro-1,2,4-triazole-3-one. Ce composé est explosif et il peut atteindre des concentrations d'environ 15 g/l dans certaines solutions telles que des effluents de conditionnement industriel issus de la fabrication d'explosifs. Ces effluents sont polluants au regard de la législation en vigueur et sont actuellement stockés en l'état en attendant un procédé de traitement approprié.

Le procédé de l'invention permet précisément de traiter de telles solutions, comprenant en particulier un composé tel que l'ONTA, c'est-à-dire un composé hétérocyclique azoté explosif comprenant au moins un groupe nitro, en convertissant ce composé hétérocyclique azoté explosif en un composé non explosif.

Dans l'exemple précité, le procédé de l'invention permet de convertir le groupe nitro de l'ONTA en groupe amino pour former le 5-amino-1,2,4-triazole-3-one.

De plus, le 5-amino-1,2,4-triazole-3-one formé est un composé valorisable, car il peut être un précurseur pour la synthèse de composés ayant par exemple une activité antivirale tels que la ribavirine, et la pirazomicine, une activité inhibitrice des NO-synthases (NO.S.) tels que la 3-amino-1,2,4-triazine et l'aminoguanidine, une activité herbicide telle que la 5-amino-1,2,4-triazole, une activité antiparasitaire et une activité cytotoxique.

Le procédé de l'invention permet donc, dans certains cas, de transformer un composé non valorisable en un composé valorisable.

Un autre aspect du procédé de l'invention est la synthèse de l'amine hétérocyclique 5-amino-1,2,4-triazole-3-one à partir du 5-nitro-1,2,4-triazole-3-one.

Le composé aromatique comportant au moins un groupe nitro peut par exemple être un cycle benzène comportant un ou plusieurs groupes nitro par exemple un composé choisi dans le groupe comprenant la 2,6-dinitroaniline, le 1,3-dinitrobenzène, la 2-nitroaniline, les acides ortho-, méta- et para-nitrobenzoiques.

Les amines aromatiques ou hétérocyliques azotés sont souvent obtenus par réduction de l'analogue nitré. Cette réduction est catalysée par diverses méthodes utilisant des agents réducteurs tels que du zinc, du fer ou de l'étain, en milieu acide ou des hydrures tel que l'hydrure d'aluminium et de lithium, ou l'hydrogénation catalytique. Cependant, lorsque les amines aromatiques sont complexes, c'est-à-dire lorsqu'elles comprennent, outre le groupement nitro, des groupements fonctionnels sensibles, de nombreuses réactions secondaires peuvent se produire sur ces groupements, diminuant ainsi le rendement de la synthèse de l'amine aromatique visée.

Par exemple, la réduction de l'ONTA par hydrogénation catalytique en présence d'oxyde de platine peut affecter en plus du groupement nitro l'aromaticité de la molécule.

Par ailleurs, les conditions expérimentales de ces méthodes de synthèse sont souvent drastiques et dangereuses.

Le document WO 96/25365 décrit un procédé de dégradation de contaminants nitroaromatiques utilisant des fibres organiques, des microorganismes et des particules de métal multivalent. La dégradation consiste en une transformation des groupes nitro en groupe amino de ces composés.

### Exposé de l'invention

La présente invention se rapporte à un procédé d'élimination au moins partielle d'au moins un composé hétérocyclique azoté, ou aromatique, comportant au moins un groupe nitro, dans une solution, ledit procédé comprenant les étapes suivantes :
- mise en contact de la solution du composé hétérocyclique azoté, ou aromatique, à éliminer dans les conditions de la revendication 1 annexée, et
- séparation du micro-organisme de la solution du composé hétérocyclique azoté, ou aromatique, dont au moins une partie des groupes nitro a été convertie en groupes amino.

Selon le procédé de l'invention, la solution de composé hétérocyclique azoté et/ou aromatique comportant au moins un groupe nitro peut contenir un seul composé ou plusieurs composés différents. Cette solution peut être un effluent de conditionnement industriel issu de la fabrication d'explosifs, un effluent industriel, des eaux souillées et plus généralement une solution comprenant des composés hétérocycliques azotés, et/ou aromatiques, comportant au moins un groupe nitro qu'il est nécessaire de traiter par conversion de groupe(s) nitro en groupe(s) amino. Cette solution sera également appelée, ci-après, solution à traiter, et le composé hétérocyclique azoté, ou aromatique, comportant au moins un groupe nitro sera également appelé, ci-après, composé hétérocyclique azoté à traiter, ou composé aromatique à traiter respectivement.

Selon le procédé de l'invention, le composé hétérocyclique azoté comportant au moins un groupe nitro peut être un triazole.

Selon le procédé de l'invention, lorsque le composé hétérocyclique azoté à traiter est un triazole, il peut être un composé de formule (I) suivante : 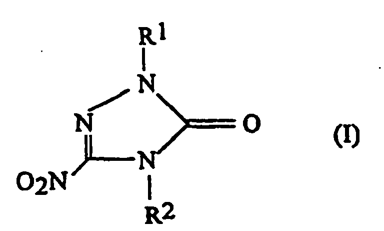
dans laquelle les groupes R¹ et R², qui peuvent être identiques ou différents, représentent H, ou un substituant choisi dans le groupe comprenant -NO₂, -OH, -COOH, -Cl, NH₂, ou un ose cyclique ou linéaire, non substitué ou substitué par un ou plusieurs groupes choisis indépendamment dans le groupe comprenant par exemple un acétyle ou un toluyle, ou un groupe alkyle, linéaire ou cyclique, comprenant de 1 à 10 atomes de carbone non substitué(s) ou substitué(s) par un ou plusieurs groupes choisis indépendamment dans le groupe comprenant -NO₂, -OH, -COOH, -Cl, NH₂. Par exemple, lorsque R₁ et/ou R₂ est un ose, il peut être le ribose, le désoxyribose, etc non substitué ou substitué.

Selon le procédé de l'invention, le composé de formule (I) peut être converti en composé de formule (II) suivante : 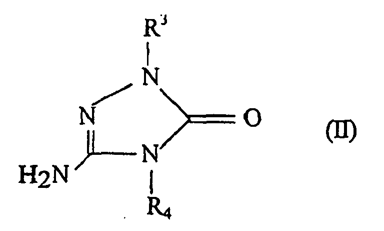
dans laquelle les groupes R³ et R⁴, qui peuvent être identiques ou différents, représentent H, un substituant choisi dans le groupe comprenant -NO₂, -OH, -COOH, -Cl, NH₂, ou un ose cyclique ou linéaire, non substitué ou substitué par un ou plusieurs groupes choisis indépendamment dans le groupe comprenant par exemple un acétyle ou un toluyle, ou un groupe alkyle, linéaire ou cyclique, de 1 à 10 atome(s) de carbone non substitué(s) ou substitué (s) par un ou plusieurs groupes choisis indépendamment parmi -NO₂, -OH, -COOH, -Cl, NH₂. Par exemple, lorsque R₁ et/ou R₂ est un ose, il peut être le ribose, le désoxyribose, le β-D-ribofuranosyl, etc...

Lorsque R¹ est un groupe -NO₂, ce groupe peut être réduit en amine correspondante et R³ peut alors être un groupe -NH₂. De même, lorsque R² est un groupe -NO₂, ce groupe peut être réduit en amine correspondante et R⁴ peut alors être un groupe -NH₂.

Le composé aromatique comportant au moins un groupe nitro à traiter peut être par exemple un mono-, di- ou trinitrobenzène, la 2-nitroaniline, les acides ortho-, méta- et paranitrobenzoïques.

Par exemple, lorsque la 2,6 dinitroaniline est traitée, on peut obtenir la 2-amino-6-nitroaniline, lorsque le 1,3 dinitrobenzène est traité, on peut obtenir le 1-amino-3-nitrobenzène, lorsque la 2-nitroaniline est traitée, on peut obtenir la 2-aminoaniline, et lorsque les acides ortho-, méta- et para-nitrobenzoïques sont traités, on peut obtenir des acides ortho-, méta- et para-aminobenzoiques respectivement.

Selon l'invention, Bacillus licheniformis peut être sélectionné par criblage de micro-organismes vivant, ou pouvant vivre, dans un effluent de conditionnement industriel issu de la fabrication d'explosifs, dans une eau souillée, et plus généralement dans un milieu, appelé ci-après milieu d'origine, comprenant des composés hétérocycliques azotés comportant de préférence au moins un groupe nitro. Par exemple, le criblage peut être réalisé à partir d'un échantillon pouvant être traité selon le procédé de l'invention, par exemple un échantillon d'un effluent de conditionnement industriel dans la fabrication d'explosifs, ou d'une eau d'épuration, comprenant des composés organiques comportant des groupes nitro, des composés aromatiques nitrés, des composés hétérocycliques azotés, etc...

Selon le procédé de l'invention, le micro-organisme capable de convertir ledit (lesdits) groupe(s) nitro en groupe(s) amino peut être sélectionné à partir d'une collection de laboratoire.

Selon le procédé de l'invention, la sélection par criblage peut être réalisée par exemple à partir d'une culture de micro-organismes vivant dans un milieu d'origine tel que ceux cités ci-dessus. Cette culture est réalisée de préférence sur un milieu de culture riche, de manière à pouvoir maintenir en vie et cultiver un maximum des micro-organismes présents dans ce milieu d'origine. D'autre part, le milieu de culture est de préférence solide afin que les micro-organismes puissent former des colonies.

Un exemple d'un milieu de culture riche pour la sélection par criblage est un milieu comprenant pour 1 litre d'eau :

| | | | |
|---|---|---|---|
| K₂HPO₄ | 1 g | | |
| KH₂PO₄ | 0, 5 g | NaNO₃ | 2 g |
| glucose | 30 g | KCl | 0,5 g |
| liqueur de maïs | 10 g | FeSO₄ | 0,02 g |
| MgSO₄ | 0,5 g | | |

Le milieu est stérilisé à 120°C pendant 20 minutes avant utilisation.

Lorsque le nombre de micro-organismes est trop important dans le milieu d'origine, la culture de ces micro-organismes sur un milieu de culture riche solide sera précédée d'une dilution d'un échantillon du milieu d'origine afin de pouvoir former des colonies isolées de ces micro-organismes.

Une analyse de la capacité de chacune des colonies formées sur le milieu riche solide à réduire des groupes nitro d'hétérocycles azotés à traiter et une analyse des caractères biochimiques de chacune de ces colonies, permettent respectivement de mettre en évidence et d'identifier les micro-organismes pouvant être utilisés selon le procédé de l'invention.

Selon l'invention, de préférence, le micro-organisme est un micro-organisme répandu dans le milieu naturel et ne présentant pas de toxicité particulière.

Selon l'invention, le micro-organisme peut, en plus de la conversion d'un ou de plusieurs groupe(s) nitro, de composés hétérocycliques azotés ou de composés aromatiques, en groupe amino, métaboliser le composé hétérocyclique azoté, ou le composé aromatique, comportant ledit(lesdits) groupe(s) amino de manière à dégrader en partie ou totalement ce composé.

Le Bacillus licheniformis préféré peut être la souche Bacillus licheniformis LCM2 déposée sous le numéro I-1915 à la CNCM (Collection Nationale de culture des Micro-organismes) tenue par l'Institut Pasteur en France.

Selon le procédé de l'invention, le micro-organisme Bacillus licheniformis, convient par exemple pour convertir le composé hétérocyclique azoté à traiter de formule (III) suivante : 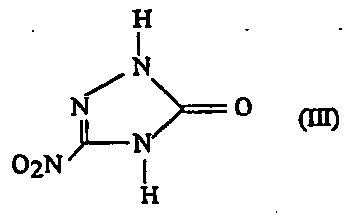 en composé hétérocyclique azoté de formule (IV) suivante :

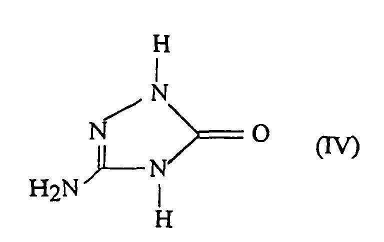

Selon le procédé de l'invention, la mise en contact d'une solution dudit composé hétérocyclique azoté à traiter avec un micro-organisme capable de réduire ledit (lesdits) groupe(s) nitro en groupe(s) amino est réalisée en mélangeant une biomasse dudit micro-organisme avec ladite solution.

La biomasse du micro-organisme peut être obtenue par culture du ou des micro-organismes sélectionnés par criblage dans un milieu de culture riche, solide ou liquide, de préférence liquide.

Un milieu riche utilisable pour développer la biomasse, lorsque le micro-organisme est Bacillus licheniformis est un milieu comprenant pour un litre d'eau :

| | | | |
|---|---|---|---|
| K₂HPO₄ | 1 g | | |
| KH₂PO₄ | 0,5 g | | |
| glucose | 30 g | NaNO₃ | 2 g |
| liqueur de maïs | 10 g | KCl | 0,5 g |
| MgSO₄ | 0,5 g | FeSO₄ | 0,02 g |

Le milieu est stérilisé à 120°C pendant 20 minutes avant utilisation.

Lorsqu'une quantité suffisante de biomasse est obtenue, et lorsque le milieu de culture est liquide, cette biomasse peut être récupérée en la compactant par centrifugation du milieu de culture.

Selon le procédé de l'invention, la biomasse peut être obtenue à partir d'un ou de plusieurs type(s) de micro-organismes capable(s) de réduire un ou des groupe(s) nitro en groupe(s) amino selon le procédé de l'invention.

La biomasse mélangée avec la solution de composé hétérocyclique azoté à traiter pour la mise en contact doit être en quantité suffisante pour permettre la conversion du (des) groupe(s) nitro du (des) composé(s) hétérocyclique(s) azoté(s) ou aromatique(s) à traiter présent(s) dans cette solution. La quantité de biomasse à utiliser peut être déterminée, par exemple, en fonction de la quantité de composés comportant des groupes nitro à convertir en groupes amino. Cette quantité de biomasse peut également être déterminée au moyen d'essais dans lesquels la quantité de conversion de groupes nitro d'un composé hétérocyclique azoté présent dans une solution est mesurée en fonction de la quantité de biomasse de micro-organisme mélangée à cette solution, la concentration en composés hétérocycliques azotés à traiter et la durée des essais étant identiques pour chaque essai.

La biomasse peut être mélangée à la solution de composé hétérocyclique azoté à traiter au moyen d'un agitateur classique, par exemple agitateur rotatif, et à une vitesse d'agitation pouvant aller de 50 à 250 rpm, par exemple de 100 rpm.

L'agitation peut être maintenue pendant toute la durée de mise en contact entre la biomasse de micro-organisme et le composé hétérocyclique azoté à traiter de manière à optimiser cette mise en contact. Le mélange biomasse et solution du composé hétérocyclique azoté à traiter est également appelé ci-après mélange d'incubation.

De préférence, selon le procédé de l'invention, la solution du composé hétérocyclique azoté comportant au moins un groupe nitro, ou le mélange d'incubation, peut être neutralisé à une valeur de pH permissif pour la conversion du(des) groupe(s) nitro en groupe(s) amino.

Une série d'essais consistant à mesurer la quantité de composés comportant des groupes nitro convertis dans un mélange d'incubation en fonction du pH peut permettre de déterminer une plage de pH permissif pour cette conversion, c'est-à-dire favorable à cette conversion.

Le pH de la solution du composé hétérocyclique azoté comportant au moins un groupe nitro, et/ou du mélange d'incubation, est neutralisé à une valeur comprise dans la plage de pH allant de 5,5 à 8 pour la mise en contact avec le micro-organisme.

D'autre part, lorsque le composé hétérocyclique azoté à traiter est l'ONTA, l'amine formée est stable et ne subit aucune transformation ultérieure tant que le pH du mélange d'incubation est maintenu à environ 6.

Lorsque la solution du composé hétérocyclique azoté à traiter, ou le mélange d'incubation, est à un pH non permissif acide, le pH peut être ajusté à une valeur permissive pour la conversion du composé hétérocyclique azoté à traiter au moyen d'une base, cette base pouvant être en solution ou solide. De préférence, la base est solide, de manière à ne pas diluer la solution de composé hétérocyclique azoté à traiter, elle peut par exemple être avantageusement sous forme de pastilles. Cette base peut être choisi par exemple dans le groupe comprenant NaOH, KOH.

Selon le procédé de l'invention, la mise en contact du micro-organisme avec la solution du composé hétérocyclique azoté à traiter est effectuée à une température permissive, de préférence sensiblement constante, pour une culture du micro-organisme dans un mélange d'incubation et pour une conversion des groupes nitro, c'est-à-dire une température favorable à une culture du micro-organisme et à ladite conversion. Lorsque le micro-organisme est une bactérie, en général, cette température est comprise entre 20 et 35°C, de préférence de 20 à 30°C. Lorsque la bactérie est Bacillus licheniformis, cette température est de préférence comprise dans la plage allant de 20 à 30°C et de toute préférence à environ 25°C.

La mise en contact peut être effectuée par exemple dans un fermenteur, ce dernier peut être un moyen pour maintenir la température sensiblement constante pendant la conversion des groupes nitro en groupes amino.

En outre, le fermenteur peut être équipé d'un agitateur pour maintenir une agitation lors de la mise en contact.

Selon le procédé de l'invention, un inducteur du métabolisme réducteur général, du micro-organisme utilisé, est ajouté à la solution du composé hétérocyclique azoté comportant au moins un groupe nitro, ou au mélange d'incubation, pour la mise en contact avec le micro-organisme. Cet inducteur est ajouté à la solution ou au mélange pour augmenter le métabolisme réducteur général du micro-organisme, favorisant ainsi la conversion du(des) groupe(s) nitro du composé hétérocyclique azoté en groupe(s) amino.

Une concentration optimale d'inducteur du métabolisme général réducteur peut être déterminée en réalisant des essais de conversion d'un composé hétérocyclique azoté comportant un groupe nitro en fonction de la concentration en inducteur dans un mélange d'incubation.

Cet inducteur peut être tout composé pouvant induire le métabolisme réducteur général du micro-organisme utilisé, par exemple du glucose, des polymères de glucose, de la mélasse, des hydrolysats de maïs, etc...

Lorsque l'inducteur du métabolisme réducteur général du micro-organisme choisi est le glucose, sa concentration dans la solution est de 1,5 à 10 g pour 100 ml de solution.

Par exemple, pour un litre d'une solution de composé hétérocyclique azoté d'ONTA à 15 g/l, une biomasse de Bacillus licheniformis de 90 g, une quantité de sucre de 15 g/l, un pH d'environ 6 et une température de 25°C, le procédé de l'invention permet de convertir plus de 80% de l'ONTA en amine correspondante, et même la totalité en 24 heures.

Le procédé conforme à l'invention permet donc de traiter un effluent aqueux industriel comprenant un composé hétérocyclique azoté toxique à traiter tel que par exemple l'ONTA. Lorsque le composé hétérocyclique azoté à traiter a été converti par le micro-organisme dans l'effluent à traiter en amine correspondante, cet effluent traité peut par exemple être épuré de l'amine formée par exemple par gel filtration par exemple sur Sephadex G10.

L'invention se rapporte également à un procédé de synthèse de 5-amino-1,2,4-triazole-3-one comprenant la mise en contact d'une solution de 5-nitro-1,2,4-triazole-3-one avec un micro-organisme capable de réduire le groupe 5-nitro en un groupe 5-amino.

La solution de 5-nitro-1,2,4-triazole-3 one peut être une solution aqueuse de ce composé, préparée à une concentration pouvant aller jusqu'à saturation.

Ce procédé de synthèse peut être réalisé de la même manière que le procédé de conversion d'un groupe nitro d'un composé hétérocyclique azoté à traiter, selon l'invention, tel qu'il est revendiqué.

Selon le procédé de l'invention, le micro-organisme peut être sélectionné par criblage de micro-organismes vivant ou pouvant vivre dans un effluent de conditionnement industriel issu de la fabrication d'explosifs, dans une eau souillée, et de manière générale dans un milieu, appelé dans cette description, milieu d'origine, comprenant de préférence des composés hétérocycliques azotés comportant de préférence au moins un groupe nitro.

Par exemple, le criblage peut être réalisé à partir d'un échantillon d'un milieu pouvant être traité selon le procédé de l'invention.

Selon le procédé de l'invention, le micro-organisme est du type Bacillus licheniformis.

Selon le procédé de l'invention, le Bacillus licheniformis est la souche Bacillus licheniformis LCM2 déposée sous le numéro 1-1915 à la CNCM tenue par l'Institut Pasteur en France.

Selon l'invention, lorsque la synthèse de 5-amino-1,2,4-triazole-3-one est achevée, ce dernier peut être isolé du mélange solution/biomasse par centrifugation pour éliminer la biomasse, et par purification du 5-amino-1,2,4-triazole-3-one contenu dans le surnageant de centrifugation.

Le traitement peut par exemple être réalisé par évaporation de la phase aqueuse et purification de l'extrait de 5-amino-1,2,4-triazole-3-one par des procédés classiques de purification.

L'invention se rapporte également à une souche de type Bacillus licheniformis déposée sous le numéro I-1915 à la CNCM (Collection Nationale de Culture de Micro-organismes) tenue par l'Institut Pasteur en France.

Les caractéristiques et avantages de l'invention apparaîtront mieux à la lecture de la description qui va suivre. Cette description porte sur un exemple de réalisation de l'invention donné à titre explicatif et non limitatif en référence aux figures annexées.

### Brève description des figures

La figure 1 est une représentation graphique de la conversion de l'ONTA en 5-amino-1,2,4-triazole-3-one, illustrant l'évolution de la concentration en g/l de ces deux composés en fonction du temps en heures dans un exemple de réalisation de la présente invention sur un effluent traité avec Bacillus licheniformis (souche n° I-1915 à la CNCM).
La figure 2 est une représentation graphique de la quantité en g/l d'ONTA converti en amine correspondante selon le procédé de l'invention en fonction du pH, pour 10 jours d'incubation.
La figure 3 est une représentation graphique de la quantité en g/l d'amine formée par conversion d'ONTA selon le procédé de l'invention, en fonction de la concentration en glucose, pour 7 jours d'incubation.

### Exposé détaillé d'un exemple de réalisation de l'invention

Dans cet exemple, on traite un effluent aqueux de conditionnement industriel issu de la fabrication d'explosifs.

Cet effluent aqueux industriel contient de l'ONTA de formule (III) suivante :

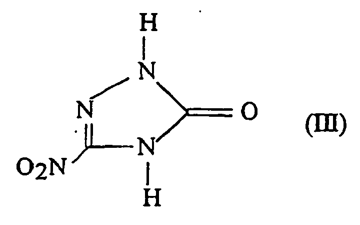

### Criblage du micro-organisme et préparation de la biomasse

Un criblage des micro-organismes présents dans l'effluent aqueux industriel précité a permis d'isoler une souche Bacillus licheniformis. Cette souche a l'avantage d'être répandue dans le milieu naturel et de ne présenter aucune toxicité. De plus, son utilisation, selon le procédé de l'invention ne nécessite aucune précaution particulière. Elle a été déposée à la CNCM sous le numéro I-1915 tenue par l'Institut Pasteur.

Pour former une biomasse, cette souche a été cultivée à 25°C pendant 48 heures dans un milieu de culture riche, liquide, de composition suivante :

| | | | |
|---|---|---|---|
| K₂HPO₄ | 1 g | | |
| KH₂PO₄ | 0,5 g | | |
| glucose | 30 g | NaNO₃ | 2 g |
| liqueur de maïs | 10 g | KCl | 0,5 g |
| MgSO₄ | 0,5 g | FeSO₄ | 0,02 g |

Le milieu est stérilisé à 120°C pendant 20 minutes.

La culture résultante a été centrifugée pour récupérer la biomasse de Bacillus licheniformis formée.

### Détermination d'un pH permissif pour la conversion de l'ONTA dans l'effluent aqueux

Une série de neuf essais E1 à E9 a été réalisée comme suit :

Dans un fermenteur, par exemple un erlenmeyer, thermostaté, dont la température a été maintenue à 25°C, comprenant un système d'agitation réglé à 100 rpm, ont été versés et mélangés :
- un litre d'effluent aqueux comprenant 15 g/l d'ONTA, et ayant un pH de 3,5,
- 90 g de biomasse de Bacillus licheniformis, et
- 15 g de glucose

pour obtenir un mélange d'incubation E ayant un pH de 3,5.

L'essai E1 était le témoin, il a été réalisé sans modifier le pH du mélange d'incubation E.

Les essais E2 à E9 ont été réalisés en neutralisant le pH du mélange d'incubation E avec des pastilles de soude de manière à obtenir pour l'essai E2 un pH 5, pour l'essai E3 un pH 5,5, pour l'essai E4 un pH 6 et ainsi de suite jusqu'à l'essai E9 dans lequel le pH a été neutralisé pour obtenir un pH 8,5.

Chaque essai a été incubé à 25°C, sous agitation à 100 rpm, pendant 10 jours. Au bout du 10ème jour, la quantité en g/l d'ONTA dans chaque mélange d'incubation a été mesurée. Le tableau 2 suivant présente les résultats obtenus pour les essais E1 à E9 :

**Tableau 2 : détermination du pH permissif pour la conversion de l'ONTA**

| Essai | E1 | E2 | E3 | E4 | E5 | E6 | E7 | E8 | E9 |
|---|---|---|---|---|---|---|---|---|---|
| pH | 3,5 | 5 | 5,5 | 6 | 6,5 | 7 | 7,5 | 8 | 8,5 |
| [ONTA] g/l au bout de 10 jours d'incubation à 25°C | 15 | 9,8 | 6,4 | 4 | 5,2 | 7,2 | 7,2 | 6,6 | 7,8 |

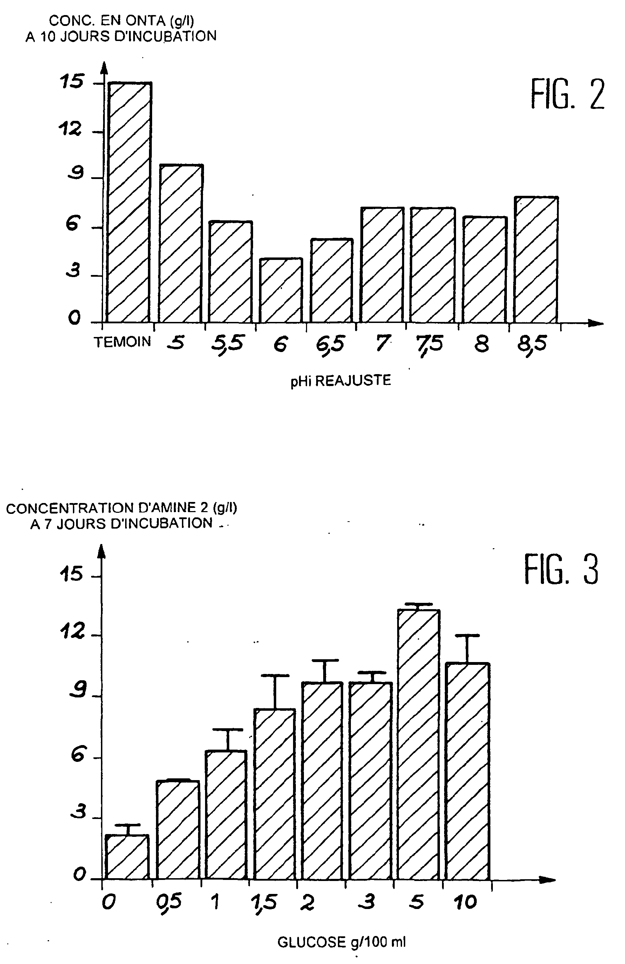

La figure 2 a été réalisée à partir des résultats du tableau 2. Elle illustre donc l'évolution de la concentration en g/l d'ONTA dans le mélange d'incubation en fonction du pH pour 10 jours d'incubation.

Ces résultats montrent que la conversion de l'ONTA en amine correspondante par Bacillus licheniformis est optimum pour un pH de 6 du mélange d'incubation, et que le pH permissif pour cette conversion est un pH supérieur à 3,5, ce pH étant compris avantageusement entre pH 5,5 et 8.

### Détermination d'une concentration optimale en inducteur du métabolisme général réducteur de Bacillus licheniformis

Une série de huit essais G1 à G8 a été réalisée à partir d'un mélange d'incubation réalisé comme suit :

Dans un fermenteur, par exemple un erlenmeyer, thermostaté, dont la température a été maintenue à 25°C, comprenant un système d'agitation réglé à 100 rpm ont été versés et mélangés :
- un litre d'effluent aqueux comprenant 15 g/l d'ONTA, ayant un pH de 3,5,
- des pastilles de soude de manière à obtenir un pH de 6, et
- 90 g de biomasse de Bacillus licheniformis

pour obtenir un mélange d'incubation G de pH égal à 6.

L'essai G1 était le témoin, il a été réalisé sans ajouter de glucose dans le mélange d'incubation G.

Les essais G2 à G8 ont été réalisés en ajoutant au mélange d'incubation G du glucose de manière à obtenir dans ces essais une concentration de glucose allant de 0,5 g (essai G2) à 10 g (essai G8) de glucose pour 100 ml de mélange d'incubation G.

Chaque essai a été incubé à 25°C, sous agitation à 100 rpm pendant 7 jours. Au bout du 7ème jour, la quantité en g/l d'amine formée par conversion de l'ONTA dans le mélange d'incubation a été mesurée. Le tableau 3 suivant présente les résultats obtenus pour les essais G1 à G8.

**Tableau 3 : détermination d'une quantité optimale de glucose pour la conversion de l'ONTA**

| Essai | G1 | G2 | G3 | G4 | G5 | G6 | G8 |
|---|---|---|---|---|---|---|---|
| [glucose] | 0 | 0,5 | 1 | 1,5 | 2 | 3 | 10 |
| g/l d'amine formée au bout de 7 jours d'incubation à-25°C | 2,1 | 4,75 | 6,25 | 8,35 | 9,65 | 9,65 | 10,6 |
| incertitude en g/l | 0,6 | 0,05 | 1,15 | 1,65 | 1,15 | 0,55 | 1,5 |

La figure 3 a été réalisée à partir des résultats du tableau 3, elle illustre donc l'évolution de la concentration en g/l d'amine formée par conversion de l'ONTA en fonction de la concentration en glucose en g/100 ml de mélange d'incubation pour 7 jours d'incubation.

Ces résultats montrent que la conversion de l'ONTA en amine correspondante par Bacillus licheniformis est maximum pour une concentration en glucose de 5 g/100 ml de mélange d'incubation.

Compte tenu d'une recherche d'un compromis entre efficacité d'induction et coût du procédé selon l'invention, les inventeurs ont choisi d'utiliser dans l'exemple qui va suivre une concentration de 15 g de glucose par litre de mélange d'incubation.

### Conversion de l'ONTA contenu dans l'effluent aqueux industriel par un micro-organisme

Dans un fermenteur thermostaté comprenant un système d'agitation, un litre d'effluent aqueux à traiter a été neutralisé à pH 6 au moyen de pastilles de soude, sous agitation à 100 rpm.

A cette solution neutralisée, ont été ajoutés 90 g de la biomasse de Bacillus licheniformis précédemment formée, et 15 g de glucose pour induire un métabolisme général réducteur chez Bacillus licheniformis. Ce mélange d'incubation a été maintenu sous agitation à 100 rpm pendant 72 heures à 25°C et la conversion du groupe nitro de l'ONTA en amine a été suivie en mesurant par chromatographie liquide haute performance l'évolution des concentrations en g/l de l'ONTA et de l'amine formée, en fonction du temps.

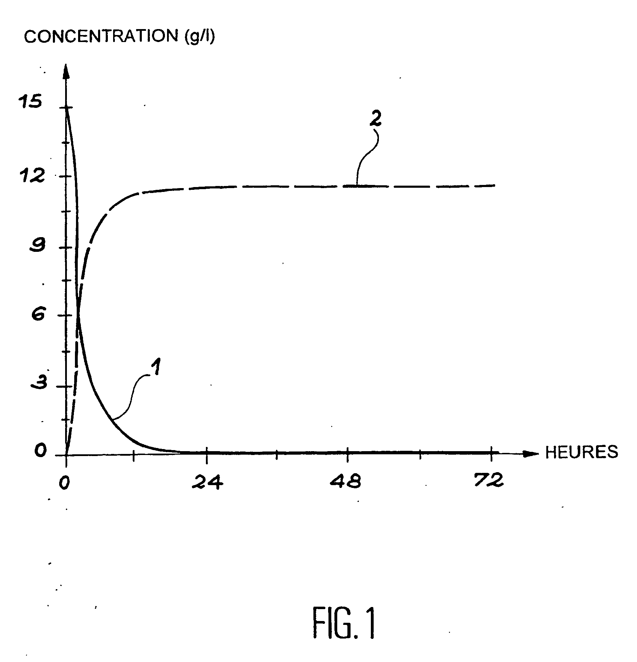

Sur la figure 1, la courbe 1 est la courbe illustrant la conversion de l'ONTA et la courbe 2 est la courbe illustrant la formation de l'amine correspondante.

Ces courbes montrent que la totalité de l'ONTA explosif contenu dans la solution initiale, a été transformée en amine non explosif en 24 heures.

D'autre part, la courbe 2 montre que l'amine formée ne subit aucune transformation ultérieure lorsque le pH est maintenu à 6.

Au bout des 72 heures, le mélange a été centrifugé à 18000 g pour séparer la biomasse de la solution. La solution obtenue a été purifiée après lyophilisation, par reprise dans du méthanol et après évaporation du méthanol, reprise dans un volume réduit d'eau pour obtenir une solution concentrée.

La purification de l'amine a été réalisée par une élution à l'eau sur une colonne de gel SEPHADEX G 10. L'amine formée étant de couleur jaune, le suivi de son élution était facilité. L'eau de l'éluant a ensuite été évaporée et l'amine récupérée sous forme d'une poudre jaunâtre présentant les caractéristiques chimiques du tableau 4 suivant :

**Tableau 4 : caractéristique de l'amine formée**

| Point de fusion | 240-295°C |
|---|---|
| RMN¹³C | (DMSO, 300 Mhz)δ : 155,1 (C=O), 147,8 (C=N) |
| IR | 3684, 3620, 3023, 2980, 2889, 2403, 1525, 1469, 1216, 1040, 920 |
| SMIE | 100 (M⁺, 83), 57 (35), 43 (100), CIMS : m/z 101 (M+H⁺) |
| Anal. | C₂H₄N₄O, Calc. C, 24 ; H, 4 ; N, 55 ; 0,16. Trouvé C, 24,6 ; H, 3, 8 ; N, 54,1 ; 0, 17,4 |

Cette amine de formule (IV) suivante : 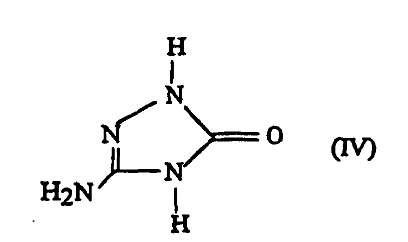 obtenue à partir de l'ONTA de l'effluent est intéressante car elle peut être valorisée.

D'autre part, la souche Bacillus licheniformis étant non toxique, l'effluent traité, dont on a extrait l'ONTA explosif transformé en amine non explosif, présentait une non toxicité telle qu'il aurait pu être relâché dans la nature sans présenter de risques majeurs pour l'environnement.

## Revendications

1. Procédé d'élimination au moins partielle d'au moins un composé hétérocyclique azoté, ou aromatique, comportant au moins un groupe nitro, dans une solution, ledit procédé comprenant les étapes suivantes :
- mise en contact de la solution du composé hétérocyclique azoté, ou aromatique, à éliminer avec Bacillus licheniformis pour convertir ledit (lesdits) groupe(s) nitro du composé hétérocyclique ou aromatique en groupe(s) amino, ladite mise en contact étant réalisée à un pH allant de 5,5 à 8, avec une biomasse de Bacillus licheniformis de 90g/l de solution, et en présence d'une concentration en glucose allant de 1,5 à 10g pour 100 ml de solution; et
- séparation du micro-organisme de la solution du composé hétérocyclique azoté ou aromatique dont au moins une partie des groupes nitro a été convertie en groupes amino.

2. Procédé selon la revendication 1, dans lequel le composé hétérocyclique comportant au moins un groupe nitro est un composé triazole.

3. Procédé selon la revendication 2, dans lequel le triazole est un composé de formule (I) suivante : 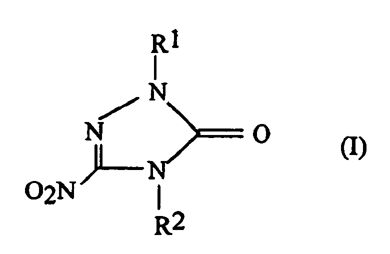
dans laquelle les groupes R¹ et R², qui peuvent être identiques ou différents, représentent H, ou un substituant choisi dans le groupe comprenant -NO₂, -OH, -COOH, -Cl, NH₂, ou un ose cyclique ou linéaire, non substitué ou substitué par un ou plusieurs groupes choisis indépendamment dans le groupe comprenant un acétyle ou un toluyle, ou un groupe alkyle, linéaire ou cyclique, comprenant de 1 à 10 atomes de carbone non substitué(s) ou substitué(s) par un ou plusieurs groupes choisis indépendamment dans le groupe comprenant -NO₂, -OH, -COOH, -Cl, NH₂.

4. Procédé selon l'une quelconque des revendications 1 à 3, dans lequel Bacillus licheniformis est sélectionné par criblage de micro-organismes pouvant vivre dans un milieu comprenant des composés hétérocycliques azotés ou aromatiques.

5. Procédé selon la revendication 1, dans lequel Bacillus licheniformis dégrade le composé hétérocyclique azoté, ou aromatique, comportant au moins un groupe nitro en groupe amino.

6. Procédé selon la revendication 1, dans lequel Bacillus licheniformis est la souche Bacillus licheniformis LCM2 déposée sous le numéro 1-1915 à la CNCM tenue par l'Institut Pasteur.

7. Procédé selon l'une quelconque des revendications 1 à 6, dans lequel le composé hétérocyclique azoté répond à la formule (III) suivante :

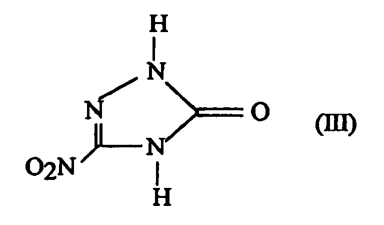

8. Procédé selon l'une quelconque des revendications précédentes, dans lequel la solution de composé hétérocyclique azoté est un effluent de conditionnement issu de la fabrication d'explosifs.

9. Procédé de synthèse de 5-amino-1,2,4-triazole-3-one à partir de 5-nitro-1,2,4-triazole-3-one par réduction du groupe 5-nitro en un groupe 5-amino, ledit procédé utilisant un procédé selon la revendication 1 précédente, dans lequel le composé hétérocyclique azoté est le 5-nitro-1,2,4-triazole-3-one.

10. Procédé selon la revendication 9, dans lequel le Bacillus licheniformis est la souche Bacillus licheniformis LCM2 déposée sous le numéro 1-1915 à la CNCM tenue par l'Institut Pasteur en France.

## Patentansprüche

1. Verfahren zur mindestens partiellen Eliminierung mindestens einer stickstoffhaltigen heterocyclischen oder aromatischen Verbindung, die mindestens eine Nitro-Gruppe trägt, aus einer Lösung, wobei das genannte Verfahren die folgenden Stufen umfasst:
- Inkontaktbringen der Lösung der stickstoffhaltigen heterocyclischen oder aromatischen Verbindung, die eliminiert werden soll, mit Bacillus licheniformis, um die genannte(n) Nitrogruppe(n) der heterocyclischen oder aromatischen Verbindung in eine oder mehrere Aminogruppen zu überführen, wobei das genannte Inkontaktbringen durchgeführt wird bei einem pH-Wert von 5,5 bis 8 mit einer Biomasse von Bacillus licheniformis von 90 g/l Lösung in Gegenwart einer Glucose-Konzentration von 1,5 bis 10 g pro 100 ml Lösung; und
- Abtrennung des Mikroorganismus von der Lösung der stickstoffhaltigen heterocyclischen oder aromatischen Verbindung, in der mindestens ein Teil der Nitrogrupppen in Aminogruppen umgewandelt worden ist.

2. Verfahren nach Anspruch 1, in dem die heterocyclische Verbindung, die mindestens eine Nitrogruppe trägt, eine Triazol-Verbindung ist.

3. Verfahren nach Anspruch 2, in dem das Triazol eine Verbindung der nachstehend angegebenen Formel (I) ist: 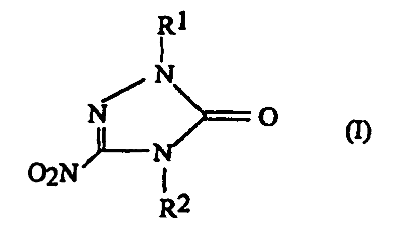 in der die Gruppen R¹ und R², die gleich oder verschieden sein können, jeweils stehen für Wasserstoff oder einen Substituenten, ausgewählt aus der Gruppe, die umfasst -NO₂, -OH, -COOH, -Cl, -NH₂ oder eine cyclische oder lineare Ose-Verbindung, die unsubstituiert ist oder substituiert ist durch eine oder mehrere Gruppen, unabhängig voneinander ausgewählt aus der Gruppe, die umfasst eine Acetyl- oder Toluyl-Gruppe, oder eine lineare oder cyclische Alkylgruppe mit 1 bis 10 Kohlenstoffatomen, das (die) unsubstituiert ist (sind) oder substituiert ist (sind) durch eine oder mehrere Gruppen, unabhängig voneinander ausgewählt aus der Gruppe, die umfasst
-NO₂, -OH, -COOH, -Cl, -NH₂.

4. Verfahren nach einem der Ansprüche 1 bis 3, in dem der Bacillus licheniformis selektioniert wird durch Auswahl von Mikroorganismen, die in einem Medium leben können, das stickstoffhaltige heterocyclische oder aromatische Verbindungen enthält.

5. Verfahren nach Anspruch 1, in dem der Bacillus licheniformis die stickstoffhaltige heterocyclische oder aromatische Verbindung, die mindestens eine Nitrogruppe trägt, zu einer Aminogruppe abbaut.

6. Verfahren nach Anspruch 1, in dem der Bacillus licheniformis der Stamm Bacillus licheniformis LCM2 ist, der bei der CNCM-Hinterlegungsstelle des Institut Pasteur unter der Nummer 1-1915 hinterlegt ist.

7. Verfahren nach einem der Ansprüche 1 bis 6, in dem die stickstoffhaltige heterocyclische Verbindung der folgenden Formel (III) entspricht:

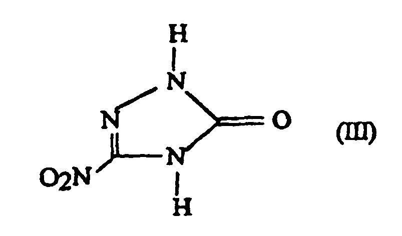

8. Verfahren nach einem der vorhergehenden Ansprüche, in dem die Lösung der stickstoffhaltigen heterocyclischen Verbindung ein Konditionierungs-Abstrom ist, der bei der Herstellung von Sprengstoffen erhalten wird.

9. Verfahren zur Synthese von 5-Amino-1,2,4-triazol-3-on aus 5-Nitro-1,2,4-triazol-3-on durch Reduktion der 5-Nitro-Gruppe zu einer 5-Amino-Gruppe, wobei bei diesem Verfahren ein Verfahren nach Anspruch 1 angewendet wird, in dem die stickstoffhaltige heterocyclische Verbindung das 5-Nitro-1,2,4-triazol-3-on ist.

10. Verfahren nach Anspruch 9, in dem der Bacillus licheniformis der Stamm Bacillus licheniformis LCM2 ist, der bei der CNCM-Hinterlegungsstelle des Institut Pasteur in Frankreich unter der Nummer I-1915 hinterlegt ist.

## Claims

1. Method for the at least partial removal of at least one nitrogen-containing heterocyclic compound or aromatic compound comprising at least one nitro group, in a solution, said method comprising the following steps:
- contacting the solution of the nitrogen-containing heterocyclic compound or aromatic compound to be eliminated with Bacillus licheniformis in order to convert said nitro group or groups of the heterocyclic or aromatic compound into amino group or groups, said contacting taking place at a pH between 5.5 and 8 with a 90 g/l solution Bacillus licheniformis biomass and in the presence of a glucose concentration ranging from 1.5 to 10 g per 100 ml of solution and
- separation of the microorganism from the solution of the nitrogen-containing or aromatic heterocyclic compound, whereof at least part of the nitro groups has been converted into amino groups.

2. Method according to claim 1, in which the heterocyclic compound comprising at least one nitro group is a triazole compound.

3. Method according to claim 2, in which the triazole is a compound with the following formula (I): 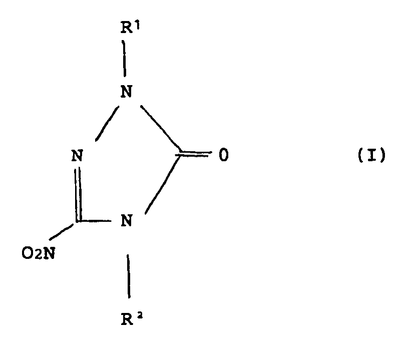
in which the R¹ and R² groups, which can be identical or different, represent H, or a substituent group chosen from within the group comprising -NO₂, -OH, -COOH, -Cl, -NH₂, or a cyclic or linear glucose unit, non-substituted or substituted by one or more groups chosen independently from within the group comprising an acetyl or a toluyl group or a linear or cyclic alkyl group, comprising from 1 to 10 atoms of carbon, non-substituted or substituted by one or more groups chosen independently from within the group comprising -NO₂, -OH, -COOH, -Cl, -NH₂.

4. Method according to any one of claims 1 to 3, in which Bacillus licheniformis is selected by screening microorganisms that are able to live in a medium comprising nitrogen-containing heterocyclic compounds or aromatic compounds.

5. Method according to claim 1, in which the Bacillus licheniformis degrades the nitrogen-containing heterocyclic compound or aromatic compound, comprising at least one nitro group into an amino group.

6. Method according to claim 1, in which the Bacillus licheniformis is the Bacillus licheniformis strain LCM2 filed under number I-1915 in the CNCM held by the Pasteur Institute.

7. Method according to any one of the claims 1 to 6, in which the nitrogen-containing heterocyclic compound corresponds to the following formula (III):

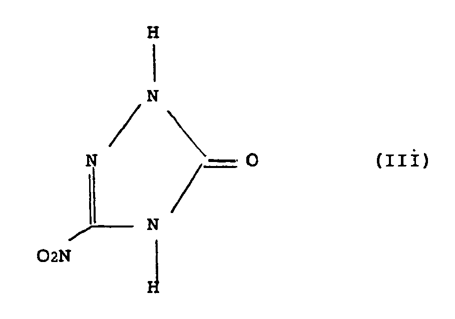

8. Method according to any one of the preceding claims, in which the solution of nitrogen-containing heterocyclic compound is a processing effluent arising from the manufacture of explosives.

9. Method for the synthesis of 5-amino-1,2,4-triazole-3-one from 5-nitro-1,2,4-triazole-3-one by reducing the 5-nitro group into a 5-amino group using a method according to claim 1, in which the nitrogen-containing heterocyclic compound is 5-nitro-1,2,4-triazole-3-one.

10. Method according to claim 9, in which the Bacillus licheniformis is the Bacillus licheniformis strain LCM2 filed under number I-1915 in the CNCM held by the Pasteur Institute in France.
